(19) Europäisches Patentamt
European Patent Office
Office européen des brevets

(11) Veröffentlichungsnummer: **0 243 776**
**A2**

(12) **EUROPÄISCHE PATENTANMELDUNG**

(21) Anmeldenummer: 87105462.3

(22) Anmeldetag: 13.04.87

(51) Int. Cl.³: C 07 D 333/38
C 07 D 409/04, C 07 D 307/54
C 07 D 307/80, A 01 N 47/18
//C07D493/04

(30) Priorität: 24.04.86 DE 3613792

(43) Veröffentlichungstag der Anmeldung: 04.11.87 Patentblatt 87/45

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Daum, Werner, Dr.
Bärenstrasse 18
D-4150 Krefeld 1(DE)

(72) Erfinder: Hänssler, Gerd, Dr.
Am Arenzberg 58a
D-5090 Leverkusen 3(DE)

(72) Erfinder: Ooms, Pieter, Dr.
Doerperhofstrasse 16
D-4150 Krefeld(DE)

(54) **Carbamoyloxythiophen-Derivate.**

(57) Neue Carbamoyloxythiophen-Derivate der allgemeinen Formel (I)

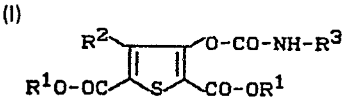

(I)

in welcher

$R^1$, $R^2$ und $R^3$ die in der Beschreibung gegebenen Bedeutungen haben und ihre Verwendung zur Bekämpfung von Schädlingen, vor allem als Fungizide.

Die neuen Carbamoyloxythiophen-Derivate der allgemeinen Formel (I) können hergestellt werden, z.B. aus geeigneten Isocyanaten mit geeigneten Hydroxythiophen-Derivaten oder z.B. aus geeigneten Chlorcarbonyloxythiophen-Derivaten mit geeigneten Aminen bzw. Aminsalzen gegebenenfalls in Gegenwart eines Lösungsmittels und gegebenenfalls in Gegenwart eines Säureakzeptors.

EP 0 243 776 A2

BAYER AKTIENGESELLSCHAFT 5090 Leverkusen, Bayerwerk
Konzernverwaltung RP
Patentabteilung Bas/Sdt/Kü-c
Ib

# Carbamoyloxythiophen-Derivate

Die vorliegende Erfindung betrifft neue Carbamoyloxythiophen-Derivate, Verfahren zu ihrer Herstellung und ihre Verwendung als Schädlingsbekämpfungsmittel, vor allem als Fungizide.

Es ist bereits bekannt, daß Thiophen-Derivate, wie z.B. das 2,5-Bis-(butoxycarbonyl)-3-methyl-4-styrylcarbonyloxythiophen und das 2,5-Bis-(ethoxycarbonyl)-3,4-bis-(benzoyloxy)-thiophen, fungizide Eigenschaften besitzen (vgl. EP 93 384, EP 32 748 und DOS 3 402 625). Besonders ist das 2,5-Bis-(isopropoxycarbonyl)-3-methyl-4-(3-methylbenzoyloxy)-thiophen zu nennen, das bekannt ist aus T. Wada et al., Proceedings of the 10th Internat. Congress of Plant Protection, Nov. 20.-25.1983, Brighton, Vol. 1, Seiten 400-407. Die Wirkungen dieser Verbindungen können unter bestimmten Bedingungen, wie z.B. bei niedrigen Aufwandmengen und Konzentrationen nicht immer ganz befriedigend sein.

Es wurden neue Carbamoyloxythiophen-Derivate der allgemeinen Formel (I)

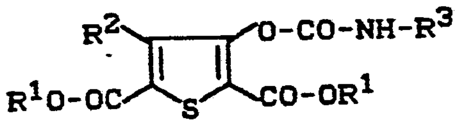
(I)

in welcher

$R^1$ für Alkyl, Alkoxalkyl, Alkylthioalkyl, Fluoralkyl, Cyanalkyl, Alkenyl, Alkinyl oder Cycloalkyl steht,

$R^2$ für Alkyl, gegebenenfalls substituiertes Furyl oder für gegebenenfalls substituiertes Phenyl steht,

$R^3$ für aliphatisches oder carbocyclisches Cyanalkyl, den Rest $-A^1-CO-N\begin{smallmatrix}R^4\\R^5\end{smallmatrix}$ oder den Rest $-A^2-CO-OR^6$ steht,

wobei

$A^1$ für einen Alkylenrest steht,

$A^2$ für einen Alkylenrest steht oder eine direkte Bindung bedeutet,

$R^4$ und $R^5$ unabhängig voneinander für Alkyl stehen oder

$R^4$ und $R^5$ gemeinsam mit dem Stickstoffatom, an dem sie stehen, einen heterocyclischen Ring bilden, der weitere Heteroatome enthalten kann und gegebenenfalls substituiert ist und

$R^6$ für Alkyl steht,

gefunden.

Weiterhin wurde gefunden, daß man die neuen Carbamoyloxythiophen-Derivate der allgemeinen Formel (I)

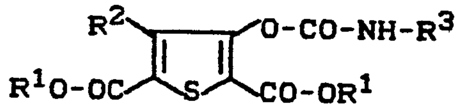

(I)

in welcher

$R^1$ für Alkyl, Alkoxalkyl, Alkylthioalkyl, Fluoralkyl, Cyanalkyl, Alkenyl, Alkinyl oder Cycloalkyl steht,

$R^2$ für Alkyl, gegebenenfalls substituiertes Furyl oder für gegebenenfalls substituiertes Phenyl steht,

$R^3$ für aliphatisches oder carbocyclisches Cyanalkyl, den Rest $-A^1-CO-N\genfrac{}{}{0pt}{}{R^4}{R^5}$ oder den Rest $-A^2-CO-OR^6$ steht,

wobei

$A^1$ für einen Alkylenrest steht,

$A^2$ für einen Alkylenrest steht oder eine direkte Bindung bedeutet,

$R^4$ und $R^5$ unabhängig voneinander für Alkyl stehen oder

$R^4$ und $R^5$ gemeinsam mit dem Stickstoffatom, an dem sie stehen, einen heterocyclischen Ring bilden, der weitere Heteroatome enthalten kann und gegebenenfalls substituiert ist und

$R^6$ für Alkyl steht,

erhält, wenn man entweder

a) ein Isocyanat der allgemeinen Formel (II)

$$OCN\text{-}R^3 \qquad (II)$$

in welcher

$R^3$ die oben angegebene Bedeutung hat,

mit Hydroxythiophen-Derivaten der allgemeinen Formel (III)

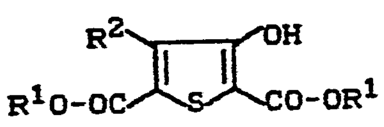

(III)

in welcher

$R^1$ und $R^2$ die oben angegebenen Bedeutungen haben,

gegebenenfalls in Gegenwart eines Lösungs- oder Verdünnungsmittels und gegebenenfalls in Gegenwart einer katalytisch wirkenden Substanz umsetzt oder wenn man

b) ein 4-Hydroxythiophen-Derivat der Formel (III) in einer ersten Stufe mit Phosgen in Gegenwart eines Chlorwasserstoff-bindenden Mittels zu einem 4-Chlorcarbonyloxythiophen-Derivat der Formel (IV)

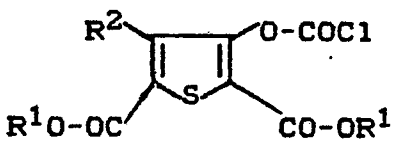

(IV)

umsetzt und dann in einer zweiten Stufe das 4-Chlorcarbonyloxythiophen-Derivat der Formel (IV) mit einem Amin der Formel (V)

$NH_2-R^3$ (V)

oder mit einem der Formel (V) entsprechenden Salz in Gegenwart eines säurebindenden Mittels umsetzt.

Weiterhin wurde gefunden, daß die neuen Carbamoyloxythiophen-Derivate der Formel (I) biologische Eigenschaften besitzen.

Überraschenderweise zeigen die erfindungsgemäßen Carbamoyloxythiophen-Derivate der Formel (I) eine bessere Wirksamkeit gegen die verschiedensten Schädlinge als die aus dem Stand der Technik bekannten Verbindungen gleicher Wirkungsrichtung oder gegenüber strukturell ähnlichen Verbindungen.

Die erfindungsgemäßen Carbamoyloxythiophen-Derivate sind durch die Formel (I) allgemein definiert. Bevorzugt sind Verbindungen der Formel (I)

in welcher

$R^1$ für geradkettiges oder verzweigtes Alkyl mit 1 bis 6 Kohlenstoffatomen, für Alkoxyalkyl oder für Alkylthioalkyl jeweils mit 1 bis 5 Kohlenstoffatomen je geradkettigen oder verzweigten Alkylteil, für Fluoralkyl mit 1 bis 4 Kohlenstoffatomen und 1 bis 9 Fluoratomen, für geradkettiges oder verzweigtes Cyanalkyl mit 1 bis 6 Kohlenstoffatomen im Alkylteil, für geradkettiges oder verzweigtes Alkenyl mit 2 bis 5 Kohlenstoffatomen, für geradkettiges oder verzweigtes Alkinyl mit 3 bis 6 Kohlenstoffatomen oder für Cycloalkyl mit 5 bis 7 Kohlenstoffatomen steht,

$R^2$ für geradkettiges oder verzweigtes Alkyl mit 1 bis 5 Kohlenstoffatomen, für Furyl, Benzofuryl oder ein- oder zweifach durch Halogen, Alkyl oder Alkoxy substituiertes Benzofuryl, oder für gegebenenfalls ein- bis fünffach, gleich oder verschieden durch Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder Halogenalkylthio mit jeweils 1 bis 4 Kohlenstoffatomen und je 1 bis 5 gleichen oder verschiedenen Halogenatomen substituiertes Phenyl steht, wobei Halogen als solches oder in den Resten wie Halogenalkyl für Fluor, Chlor, Brom oder Jod, vorzugsweise für Fluor oder Chlor, steht,

$R^3$ für geradkettiges oder verzweigtes oder für gesättigtes, cyclisches Cyanalkyl mit 1 bis 6 Kohlenstoffatomen im Alkylteil, für einen Rest

$-A^1-CO-N\genfrac{}{}{0pt}{}{R^4}{R^5}$ oder den Rest $-A^2-CO-OR^6$ steht,

wobei

$A^1$ für Alkylen mit 1 bis 6 Kohlenstoffatomen steht,

$R^4$ und $R^5$ unabhängig voneinander für Alkyl mit 1 bis 5 Kohlenstoffatomen stehen oder

$R^4$ und $R^5$ gemeinsam mit dem Stickstoffatom, an dem sie stehen, einen gesättigten oder ungesättigten, heterocyclischen Ring mit bis zu 6 Kohlenstoffatomen bilden können, der durch weitere Sauerstoffatome unterbrochen sein kann und der gegebenenfalls ein- bis fünffach, gleich oder verschieden durch Alkyl mit 1 bis 4 Kohlenstoffatomen, substituiert sein kann,

$A^2$ für Alkylen mit 1 bis 6 Kohlenstoffatomen steht oder eine direkte Bindung bedeutet und

$R^6$ für Alkyl mit 1 bis 10 Kohlenstoffatomen steht.

Besonders bevorzugt sind Verbindungen der Formel (I),

in welcher

$R^1$ für geradkettiges oder verzweigtes Alkyl mit 1 bis 5 Kohlenstoffatomen, für Alkoxyalkyl oder für Alkylthioalkyl jeweils mit 1 bis 4 Kohlenstoffatomen je geradkettigen oder verzweigten Alkylteil, für Fluoralkyl mit 1 oder 2 Kohlenstoffatomen und 1 bis 5 Fluoratomen, für geradkettiges oder verzweigtes Cyanalkyl mit 1 bis 5 Kohlenstoffatomen im Alkylteil, für geradkettiges oder verzweigtes Alkenyl mit 2 bis 4 Kohlenstoffatomen, für geradkettiges oder verzweigtes Alkinyl mit 3 bis 5 Kohlenstoffatomen oder für Cycloalkyl mit 5 oder 6 Kohlenstoffatomen steht,

$R^2$ für geradkettiges oder verzweigtes Alkyl mit 1 bis 4 Kohlenstoffatomen, für Furyl oder für gegebenenfalls ein- bis dreifach, gleich oder verschieden durch Alkyl mit 1 bis 3 Kohlenstoffatomen, Alkoxy oder Alkylthio mit je 1 bis 3 Kohlenstoffatomen, Fluor, Chlor, Nitro, Halogenalkyl, Halogenalkoxy oder Halogenalkylthio mit jeweils 1 oder 2 Kohlenstoffatomen und je 1 bis 5 gleichen oder verschiedenen Halogenatomen substituiertes Phenyl steht, wobei Halogen in den Resten wie Halogenalkyl für Fluor oder Chlor steht,

$R^3$ für geradkettiges oder verzweigtes oder für gesättigtes, cyclisches Cyanalkyl mit 1 bis 6 Kohlenstoffatomen im Alkylteil, für einen Rest

$-A^1-CO-N\begin{matrix} \diagup R^4 \\ \diagdown R^5 \end{matrix}$ oder den Rest $-A^2-CO-OR^6$ steht,

wobei

$A^1$ für Alkylen mit 1 bis 5 Kohlenstoffatomen steht,

$R^4$ und $R^5$ unabhängig voneinander für Alkyl mit 1 bis 3 Kohlenstoffatomen stehen oder

$R^4$ und $R^5$ gemeinsam mit dem Stickstoffatom, an dem sie stehen, einen gesättigten oder ungesättigten, heterocyclischen Ring mit bis zu 6 Kohlenstoffatomen bilden können, der durch ein weiteres Sauerstoffatom unterbrochen sein kann und der gegebenenfalls ein- bis dreifach, gleich oder verschieden durch Methyl oder Ethyl substituiert sein kann,

$A^2$ für Alkylen mit 1 bis 5 Kohlenstoffatomen steht oder eine direkte Bindung bedeutet und

$R^6$ für Alkyl mit 1 bis 8 Kohlenstoffatomen steht.

Insbesondere seien Verbindungen der Formel (I) genannt,

in welcher

$R^1$ für Methyl, Ethyl, n- oder iso-Propyl, 2,2-Dimethylpropyl, 2-Methoxyethyl, 2-Ethoxyethyl, Methoxypropyl, 2-Methylthioethyl, 2-Ethylthioethyl, 2,2,2-Trifluorethyl, Cyanmethyl, 2-Cyanethyl, Allyl, Methallyl, 3-Propinyl, 1,1-Dimethyl-3-propinyl oder Cyclopentyl steht,

$R^2$ für Methyl, Ethyl, n-Propyl, iso-Propyl, n-Butyl, sek.-Butyl, iso-Butyl, tert.-Butyl, Furyl, Phenyl oder für ein- bis dreifach, gleich oder verschieden durch Methyl, Ethyl, Fluor und Chlor substituiertes Phenyl steht,

$R^3$ für geradkettiges oder verzweigtes Cyanalkyl mit 2 bis 6 Kohlenstoffatomen im Alkylteil, für gesättigtes, cyclisches Cyanalkyl mit 5 oder 6 Kohlenstoffatomen im Ring, für den Rest

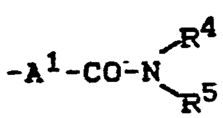 oder den Rest $-A^2-CO-OR^6$ steht,

wobei

$A^1$ für Alkylen mit 1 bis 5 Kohlenstoffatomen steht,

$R^4$ und $R^5$ unabhängig voneinander für Methyl oder Ethyl stehen oder

$R^4$ und $R^5$ zusammen mit dem Stickstoffatom, an den sie stehen, einen 6-Ring bilden, der durch ein Sauerstoffatom unterbrochen sein kann und der gegebenenfalls ein- bis dreifach, gleich oder verschieden durch Methyl oder Ethyl substituiert sein kann,

$A^2$ für Alkylen mit 1 bis 5 Kohlenstoffatomen steht oder eine direkte Bindung bedeutet und

$R^6$ für Alkyl mit 1 bis 8 Kohlenstoffatomen steht.

Besonders hervorzuheben sind Verbindungen der Formel (I),

in welcher

$R^1$ für Methyl, Ethyl, Isopropyl, n-Propyl, sek.-Butyl, 2,2-Dimethylpropyl oder Cyclopentyl steht,

$R^2$ für Methyl, Isopropyl, Furyl oder Phenyl steht,

$R^3$ für 1-Cyan-1-methylethyl, 2-Cyanethyl, 5-Cyanpentyl oder für den Rest $-A^2-COOR^6$ steht, wobei

$A^2$ eine direkte Bindung oder einen Pentylenrest bedeutet und

$R^6$ Methyl, Ethyl, Isopropyl, Butyl, 2,2-Dimethylpropyl, 2-Methylhexyl oder Octyl bedeutet.

Verwendet man als Ausgangsstoffe zur Herstellung der erfindungsgemäßen Verbindungen der Formel (I) nach Verfahren a) z.B. 2,5-Bis-(ethoxycarbonyl)-3-phenyl-4-hydroxythiophen und 1-Cyan-1-methylpropylisocyanat und Triethylendiamin als Katalysator so kann der Reaktionsverlauf durch das folgende Formelschema wiedergegeben werden:

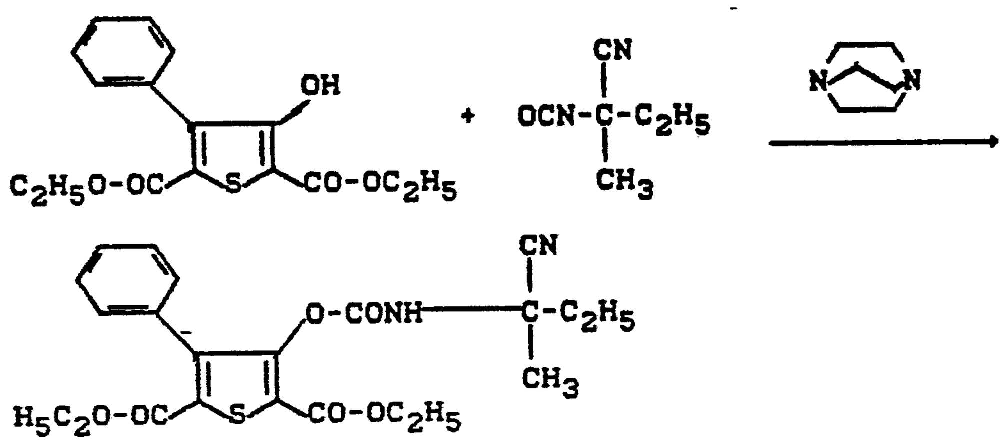

Verwendet man als Ausgangsstoffe zur Herstellung der erfindungsgemäßen Verbindungen der Formel (I) nach Verfahren b) 2,5-Bis-(n-propoxycarbonyl)-3-methyl-4-chlorcarbonyloxy-thiophen und 1-Cyan-1-amino-cyclohexan, so kann der Reaktionsverlauf durch das folgende Formelschema wiedergegeben werden:

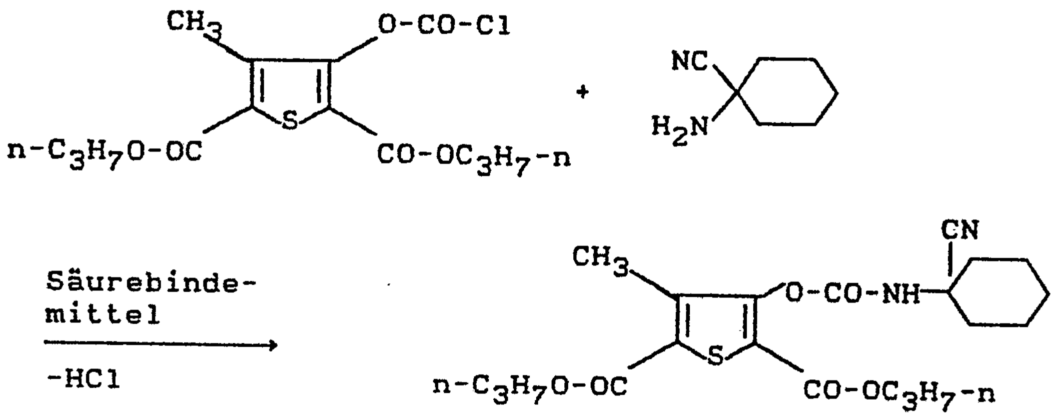


Die als Ausgangsstoffe zur Durchführung des erfindungsgemäßen Verfahrens a) benötigten Isocyanate sind durch die Formel (II) allgemein definiert. Die Verbindungen sind überwiegend bekannt und können nach bekannten Verfahren hergestellt werden. Die Isocyanate können z.B. hergestellt werden durch Umsetzen der Aminoverbindungen mit Phosgen (vgl. z.B. Liebigs Ann. Chem. 562, 75-136, (1949)), durch Umsetzen von Chlorcarbonylisocyanat mit Alkoholen bzw. mit Aminen (vgl. DOS 1 913 273) sowie durch thermische Spaltung der Phenoxycarbonylamino-Verbindungen.

Zu nennen sind hier insbesondere:

2-Cyanethyl-isocyanat, 1-Cyan-1-methylethyl-isocyanat, 1-Cyan-1-methyl-propylisocyanat, 1-Cyan-1-ethyl-propyl-isocyanat, 1-Cyan-cyclopent-1-yl-isocyanat, 1-Cyan-cyclohex-1-ylisocyanat, 3-Cyanpropyl-isocyanat, 5-Cyanpentylisocyanat, 6-Cyanhexyl-isocyanat, N-Morpholinocarbonylmethylisocyanat, 1-(N,N-Diethylaminocarbonyl)-ethylisocyanat, N-Pyrrolidino-carbonylethylisocyanat, N-Piperidino-carbonyl-propylisocyanat, N,N-Dimethylaminocarbonyl-pentylisocyanat, Methoxycarbonylmethyl-isocyanat,

Ethoxycarbonylmethyl-isocyanat, Butoxycarbonylethyl-isocyanat, Isobutoxycarbonylethyl-isocyanat, 1-Methoxycarbonyl-1-methyl-ethyl-isocyanat, 1-Propoxycarbonyl-1-methyl-ethyl-isocyanat, 1-Ethoxycarbonyl-1-ethyl-ethyl-isocyanat, 1-Isobutoxycarbonyl-1-ethyl-ethyl-isocyanat, Methoxycarbonyl-propyl-isocyanat, Methoxycarbonyl-pentyl-isocyanat, Isopropoxycarbonyl-pentyl-isocyanat, sek.-Butyloxycarbonyl-pentyl-isocyanat, Allyloxycarbonylpentyl-isocyanat, Propargyloxycarbonyl-pentyl-isocyanat, 2-Ethoxycarbonyl-2-ethyl-butyl-isocyanat, Butoxycarbonyl-pentyl-isocyanat, 2,2-Dimethylpropyloxycarbonylpentyl-isocyanat, Methoxycarbonylisocyanat, Ethoxycarbonylisocyanat, 2,2-Dimethylpropyloxycarbonyl-isocyanat, 2-Ethylhexyloxycarbonyl-isocyanat und Octyloxycarbonyl-isocyanat.

Weiterhin werden für die Umsetzung nach Verfahren a) zu den erfindungsgemäßen Verbindungen 3-Hydroxythiophen-Derivate benötigt, die durch die Formel (III) allgemein definiert sind. Die Ausgangsverbindungen der Formel (III) sind teilweise bekannt, können aber nach generell bekannten Verfahren hergestellt werden, so z.B. aus Thiodiessigsäureestern und 2-Oxocarbonsäureestern unter alkalischen Bedingungen, z.B. unter der Einwirkung von Kalium-tert.-butanolat, und an die Kondensation anschließende Behandlung mit einer Säure (vgl. EP 93 384 und DAS 1 020 641). Die Reaktion kann durch das folgende Schema veranschaulicht werden:

$R^2-CO-CO-OR + R^1O-OC-CH_2-S-CH_2-CO-OR^1 \longrightarrow$

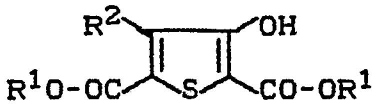

(III)

$R^1$ und $R^2$ haben die oben angegebenen Bedeutungen.

Zu nennen sind im einzelnen: 3-Methyl-4-hydroxythiophen-2,5-dicarbonsäure-methyl-, -ethyl-, -isopropyl-, -2,2-dimethylpropyl-, -cyanmethyl-, -2-cyanethyl-, -1-cyan-1-methylethyl-, -2,2,2-trifluorethyl-, -2-methoxyethyl-, -2-butylthioethyl-, -2-ethyl-thioethyl-, -allyl-, -methylallyl-, -propargyl- und -1,1-dimethylpropargyl-ester; 3-Ethyl-, 3-Propyl-, 3-Isopropyl-, 3-Butyl- und 3-tert.-Butyl-4-hydroxy-thiophen-2,5-dicarbonsäure-2,2,2-trifluorethylester; 3-(Fur-3-yl)-4-hydroxy-thiophen-2,5- dicarbonsäuremethylester; 3-Phenyl-, 3-(4-Chlorphenyl)-4- hydroxy-thiophen-2,5-dicarbonsäurecyclopentyl-ester.

Diejenigen Verbindungen der Formel III, in denen $R^2$ ein gegebenenfalls substituierter Furylrest ist, sind bisher nicht bekannt. Es handelt sich um Verbindungen der allgemeinen Formel (IIIa)

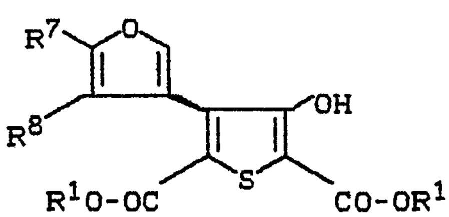

(IIIa)

worin $R^1$ die oben angegebene Bedeutung hat und $R^7$ und $R^8$ Wasserstoff bedeuten oder gemeinsam mit dem Furylrest, an den sie gebunden sind, einen Benzofurylrest der Formel (VII)

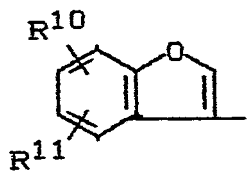

(VII)

darstellen, worin $R^{10}$ und $R^{11}$ unabhängig voneinander Wasserstoff, Halogen, Alkyl oder Alkoxy bedeuten können.

Man erhält die Verbindungen der Formel (IIIa), indem man einen Thiodiessigsäureester mit einem 2-(Fur-3-yl)-2-oxo-essigsäureester in Gegenwart von Kalium-tert.-butanolat umsetzt und anschließend in Säure gießt.

Die hierbei eingesetzten ebenfalls neuen 2-(Fur-3-yl)-2-oxo-essigsäureester der allgemeinen Formel (VIII)

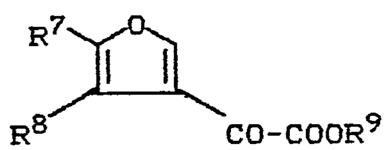

(VIII)

in denen $R^7$ und $R^8$ die oben angegebenen Bedeutungen haben und $R^9$ für gegebenenfalls durch Halogen oder Alkoxy substituiertes Alkyl steht,

erhält man, wenn man Fur-3-yl-glykolsäureester der allgemeinen Formel (VI)

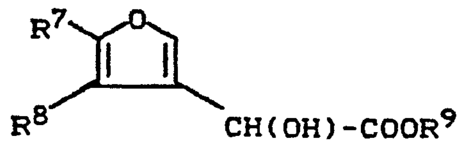

(VI)

in welcher $R^7$, $R^8$ und $R^9$ die oben genannten Bedeutungen haben, mit Oxidationsmitteln in einem inerten organischen Lösungsmittel umsetzt.

Besonders geeignet sind Verbindungen der Formel (VI), in denen

$R^7$ und $R^8$ für Wasserstoff stehen oder gemeinsam mit dem Furylrest, an den sie gebunden sind, einen Benzofuryl-rest der Formel VII darstellen, in welchen $R^{10}$ und $R^{11}$ unabhängig voneinander Wasserstoff oder Methoxy bedeuten und

$R^9$ für n-Butyl steht.

Verwendet man als Ausgangsstoffe zur Herstellung der Verbindungen der Formel (VIII) beispielsweise Fur-3-yl-glykolsäure-trifluorethylester und Chromtrioxid, so kann der Reaktionsverlauf durch das folgende Reaktionsschema wiedergegeben werden.

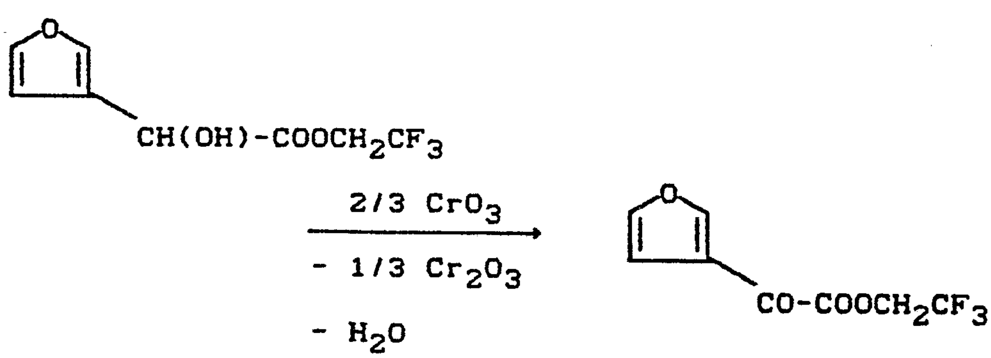

Die als Ausgangsstoffe zur Durchführung des Verfahrens benötigten Fur-3-yl-glykolsäureester, durch die Formel (VI) allgemein definiert, sind teilweise bekannt (J. Org. Chem. 42, 1089 (1977)). Man erhält sie, wenn man photochemisch Glyoxylsäureester an Furane addiert und anschließend die 2,7-Dioxabicyclo[3.2.0]hept-3-ene mit Säure isomerisiert.

Zu nennen sind hier Fur-3-yl-glykolsäure-methyl-, -ethyl-, -propyl-, -isopropyl-, -butyl-, sek.-butyl-, -isobutyl-, -n-amyl-, -trifluorethyl-, -1-trifluormethylethyl-, -methoxyethyl-, -2-ethoxy-propyl- und -butoxyethylester; 7-Methoxybenzofur-3-yl-glykol-säurebutylester, 6-Methyl- bzw. 6-Chlorbenzofur-3-yl-glykol-säurepropylester sowie Benzofur-3-yl-glykolsäureethyl- und butylester.

Noch unbekannt sind die Benzofur-3-ylglykolsäureester der allgemeinen Formel (VIa)

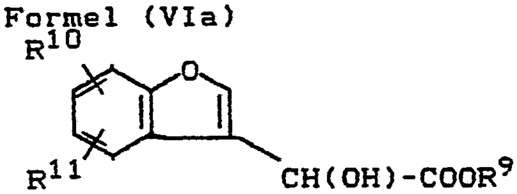

(VIa)

worin $R^9$, $R^{10}$ und $R^{11}$ die obengenannten Bedeutungen haben und deren Vorstufen.

Sie werden erhalten, wenn man Glyoxylsäureester an Benzofuran oder an die substituierten Benzofurane photochemisch in einem für photochemische Reaktionen inerten Lösungsmittel addiert und die resultierenden 2,7-Dioxabicyclo[3.2.0]hept-3-ene mit Säure isomerisiert.

Setzt man gemäß dieser Arbeitsweise beispielsweise Glyoxylsäureethylester und 7-Methylbenzofuran ein, so kann der Reaktionsverlauf durch das folgende Reaktionsschema wiedergegeben werden.

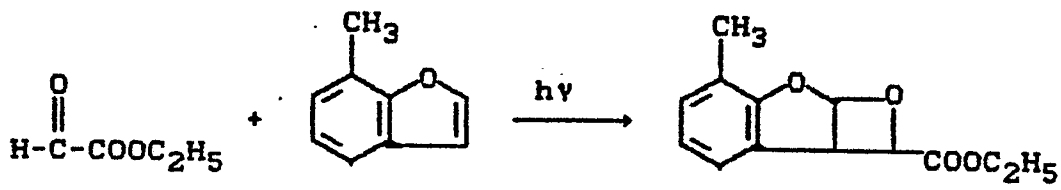


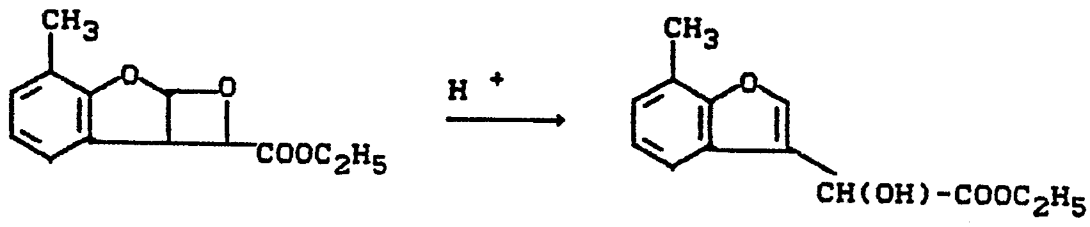


Die Photoadditionen werden unter einer inerten Atmosphäre, wie beispielsweise Stickstoff oder Edelgas, ausgeführt. Als Verdünnungsmittel kommen alle für photochemische Reaktionen inerten Lösungsmittel in Frage. Zu nennen sind hier beispielsweise Benzol, Toluol, Dioxan, Acetonitril oder 1,2-Dimethoxyethan.

Die Isomerisierungen der 2,7-Dioxabicyclo[3.2.0]hept-3-ene werden in inerten organischen Lösungsmitteln, beispielsweise Diethylether, Methylenchlorid oder Dioxan in großer Verdünnung vorgenommen. Bevorzugt sind Lösungen von 0,001 bis 1 Mol 2,7-Dioxabicyclo[3.2.0]hept-3-en pro Liter Lösungsmittel. Als Säurekatalysatoren kommen protische Säuren, vorzugsweise p-Toluolsulfonsäure, Benzolsulfonsäure und Methansulfonsäure in Frage. Infolge von Nebenreaktionen ist es unwirtschaftlich, konzentriertere Lösungen zu verarbeiten.

Für die Herstellung der Fur-3-yl-glyoxylsäureester aus den Fur-3-yl-glykolsäureestern können als Oxidationsmittel die Reagenzien verwendet werden, die einen sekundären Alkohol zu einem Keton oxidieren (siehe J. March, Advanced Organic Chemistry, McGraw Hill Kogakusha Ltd. 1977, S. 1082), vorzugsweise Chromtrioxid, Bleitetraacetat und Mangandioxid. Als Verdünnungsmittel kommen inerte Lösungsmittel wie Benzol, Toluol, Essigsäure, Methylenchlorid oder auch Pyridin und Chinolin in Frage.

Als Verdünnungsmittel kommen für das Verfahren a) zur Herstellung der Verbindungen der allgemeinen Formel (I) alle gegenüber den Reaktionspartnern inerten organischen Lösungsmittel in Frage; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Als Katalysatoren für die Umsetzung der Isocyanate mit den Hydroxythiophenen können tert.-Amine, wie Triethylamin, Triethylendiamin, Pyridin, 4-Dimethylaminopyridin oder auch Bleinaphthenat und Dibutylzinndioxid angewendet werden.

Die Reaktionstemperaturen und die Reaktionsdauer werden durch die Aktivität der Ausgangsprodukte bestimmt. Im allgemeinen arbeitet man zwischen 20°C und 120°C, vorzugsweise zwischen 60°C und 100°C.

Es kann erforderlich sein, einen Überschuß des Isocyanats der Formel (II) in die Reaktion einzusetzen.

Nach dem Verfahren b) wird in erster Stufe eine Mischung, bestehend aus einem 4-Hydroxythiophen-Derivat der Formel (III), einem tert.-Amin, wie z.B. Chinolin, Dimethylbenzylamin, Dimethylanilin, Ethyldicyclohexylamin, Ethyl-diisopropylamin, Picolin, Pyridin oder Triethylamin, und einem inerten organischen Lösungsmittel, wie z.B. Methylenchlorid, Chloroform, Chlorbenzol, Ethylacetat, Toluol oder Xylol, zu überschüssigem Phosgen, gelöst in dem inerten organischen Lösungsmittel, gegeben. Dabei arbeitet man im allgemeinen zwischen -50° C und +80° C, vorzugsweise bei 0° C bis +30° C.

Nach Isolierung des 3-Chlorcarbonyloxythiophen-Derivats der Formel (IV) wird dieses mit einem Amin der Formel (V) oder mit einem Salz des Amins der Formel (V) umgesetzt, wobei man aus wirtschaftlichen Gründen die entstehende Säure an einem tert.-Amin bindet. Wird ein Aminsalz in die Reaktion eingeführt, ist die doppelte Menge des tert.-Amins einzusetzen. Für diese Umsetzung kommen im Prinzip mit Wasser mischbare oder nicht mischbare Lösungsmittel wie vorstehend aufgeführt, in Frage. Im allgemeinen arbeitet man bei Temperaturen entsprechend der Phosgenierungsreaktion.

Je nach Arbeitsbedingungen fallen die erfindungsgemäßen Wirkstoffe kristallin aus oder sie bleiben im organischen Lösungsmittel gelöst und können dann nach Auswaschen der Lösung mit Wasser, durch vorsichtiges Einengen der Lösung oder durch Zugabe wenig polarer organischer Lösungsmittel, wie Cyclohexan, Dibutylether, Diisopropylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie auch durch Zugabe von Wasser ausgefällt werden.

Beide Verfahren werden im allgemeinen bei Normaldruck durchgeführt.

Die Aminsalze der Formel (V) können als Säurekomponente beispielsweise enthalten Chlorwasserstoffsäure, Bromwasserstoffsäure und Fluorwasserstoffsäure, insbesondere Chlorwasserstoffsäure, ferner Salpetersäure, Schwefelsäure, Essigsäure, Fumarsäure, Methansulfonsäuren, Benzolsulfonsäuren oder Toluolsulfonsäure.

Die erfindungsgemäßen Verbindungen zersetzen sich zum Teil bei höherer Temperatur; in diesen Fällen können die Schmelzpunkte nur mit geringer Genauigkeit oder überhaupt nicht ermittelt werden. Das Vorliegen bestimmter Strukturelemente ist aus den NMR-Spektrum ersichtlich.

Die erfindungsgemäßen Wirkstoffe weisen eine starke biologische Wirkung auf und können zur Bekämpfung von unerwünschten Schädlingen praktisch eingesetzt werden. Die Wirkstoffe sind u.a. für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea

(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder
Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die neuen Wirkstoffe entfalten eine besonders gute Wirksamkeit gegen Erreger von Pilzinfektionen im Reis, außerdem sind sie gut wirksam gegen Omyceten, Erreger des Apfelschorfs und zeigen bei entsprechender Anwendung und Konzentration z.B. auch gute Wirkungen gegen Getreidekrankheiten.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum,

Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

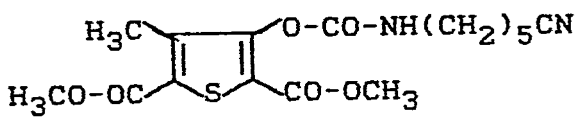

11,5 g 2,5-Bis-(methoxycarbonyl)-3-methyl-4-hydroxythiophen, 20 ml wasserfreies Acetonitril, 6,9 g 5-Cyanpentylisocyanat und 20 mg Triethylendiamin werden 4 h auf 78° C gehalten. Acetonitril wird im Vakuum abgedampft und der Abdampfrückstand mit Diisopropylether behandelt. Das Kristallisat wird abgetrennt, mit Diisopropylether gewaschen und bei 60° C/0,1 mbar getrocknet.

Ausbeute: 15,6 g 2,5-Bis-(methoxycarbonyl)-3-methyl-4-(5-cyanpentylaminocarbonyloxy)-thiophen

Schmelzpunkt: 74° C.

### Beispiel 2

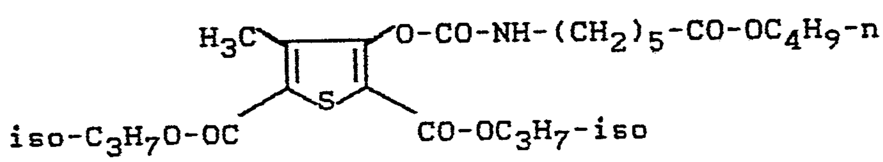

10,0 g 2,5-Bis-(isopropoxycarbonyl)-3-methyl-4-hydroxythiophen, 20 ml wasserfreies Acetonitril, 15 g Butoxycarbonylpentylisocyanat (Kp. 149-151° /13mbar), 100 mg Triethylendiamin werden 18 h auf 100° C gehalten. Es wird mit 20 ml Acetonitril verdünnt. Die Reaktionsmischung wird

dreimal mit je 50 ml Ligroin ausgeschüttelt, um das überschüssige Butoxycarbonyl-pentylisocyanat zu entfernen. Die Acetonitril-Phase wird im Vakuum eingedampft. Der Abdampfrückstand wird bei 100° C/0,1 mbar getrocknet. Man erhält 16,3 g 2,5-Bis-(isopropoxycarbonyl)-3-methyl-4-[5-(butyloxycarbonyl)-pentylaminocarbonyloxy]-thiophen als viskose Masse.

$^{1}$H-NMR 80 MHz $CDCl_3$ δ-Werte
OCH 2H m 5,0-5,5 ppm; $OCH_2$ 2H t 4,13 ppm;
N-$CH_2$ 2H m 3,1-3,5 ppm; hetar-$CH_3$ s 2,39 ppm.

Auf analoger Weise werden folgende Verbindungen der Formel (I) erhalten:

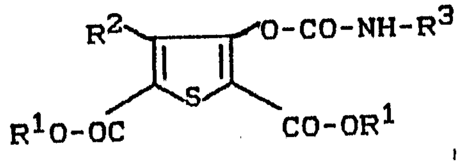

(I)

| Beispiel Nr. | $R^1$ | $R^2$ | $R^3$ | Physikalische Eigenschaften (F.= Schmelzpunkt) |
|---|---|---|---|---|
| 3 | $-CH_3$ | $(CH_3)_2CH-$ | $-(CH_2)_5-CN$ | F. 76° C aus Diisopropylether |
| 4 | $-CH_3$ | $(CH_3)_2CH-$ | $-(CH_2)_5-COOC_4H_9-n$ | viskose Masse |
| 5 | $-CH_3$ | $C_6H_5-$ | $-(CH_2)_5-CN$ | viskose Masse |
| 6 | $-C_2H_5$ | $H_3C-$ | $-(CH_2)_5-CN$ | F. 90° C aus Diisopropylether |
| 7 | $-C_3H_7-n$ | $H_3C-$ | $-(CH_2)_5-CN$ | zäh viskos |
| 8 | $-CH(CH_3)_2$ | $H_3C-$ | $-(CH_2)_5-CN$ | F. 72° C aus Diisopropylether |
| 9 | $-CH(CH_3)C_2H_5$ | $H_3C-$ | $-(CH_2)_5-CN$ | viskose Masse |
| 10 | $-CH_2C(CH_3)_3$ | $H_3C-$ | $-(CH_2)_5-CN$ | viskose Masse |
| 11 | $-CH(CH_3)_2$ | $H_3C-$ | $-(CH_2)_5-COOC_4H_9-n$ | viskose Masse |
| 12 | $-CH_3$ | 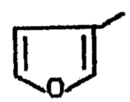 | $-(CH_2)_5-CN$ | F. 97° C aus Aceton/Diisopropylether |
| 13 | $-C_2H_5$ | $H_3C-$ | $-C(CH_3)_2-CN$ | F. 108° C aus Toluol/Petrolether |
| 14 | $-C_4H_9$ | $H_3C-$ | $-(CH_2)_5-CN$ | F. 61° C aus Dibutylether |

Beispiel 14

(Vorprodukt zur Verbindung des Beispiels 12)

2,5-Bis-(methoxycarbonyl)-3-fur-3-yl-4-hydroxythiophen.

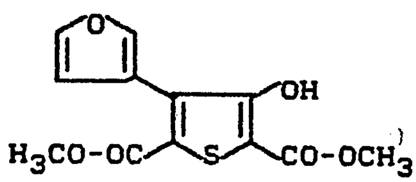

Eine Mischung aus 26 g Thiodiessigsäuredimethylester und 32 g 2-(Fur-3-yl)-2-oxo-essigsäurebutylester wird in 30 Minuten bei 64-68°C zu einer Mischung aus 100 ml Methylalkohol und 39,9 g Kalium-tert.-butanolat getropft. Die Reaktionsmischung wird noch 3 h auf dieser Temperatur belassen. Anschließend gießt man auf eine Mischung aus 179 g Eis und 58 g konzentrierter Salzsäure. Die entstehenden Kristalle werden abgetrennt, mit Eiswasser chloridfrei gewaschen und bei 60°C/0,1 mbar getrocknet. Ausbeute: 27,9 g. Man kristallisiert aus 250 ml Methylalkohol um und erhält 22 g 2,5-Bis-(methoxycarbonyl)-3-fur-3-yl-4-hydroxythiophen mit einem Schmelzpunkt von 102°C.

Beispiel 15

(Vorprodukt zur Verbindung des Beispiels 14)

2-(Fur-3-yl)-2-oxo-essigsäurebutylester

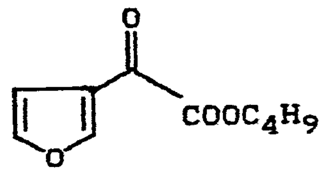

Eine Mischung aus 39.6 g Fur-3-yl-glykolsäurebutylester und 88.6 g Bleitetraacetat in 400 ml abs. Benzol wird 8 h zum Rückfluß erhitzt. Nach Abkühlen auf Raumtemperatur filtriert man die Salze ab, wäscht mit Benzol nach und schüttelt die vereinigten Benzolphasen mit einer 10 % Natriumbicarbonat-Lösung und einer gesättigten Kochsalz-Lösung aus. Nach Trocknen über Natriumsulfat, Abfiltrieren und Einengen wird der ölige Rückstand i.Vak. destilliert. Man erhält 32.7 g (83 %) Öl, Sdp. 80°C (0,3 Torr), $n_D^{20}$ = 1,477.

$^1$H-NMR (80 MHz, $CDCl_3$, δ-Werte): 8.53 (s, 1H); 7.50 (t, 1H, J = 1,5 Hz); 6,90 (d, 1H, J = 1,5 Hz); 4,35 (t, 2H, J = 7 Hz); 2.00 - 1.10 (m, 4H); 0,97 (t, 3H, J = 7 Hz)
IR (Kap. Film, v in $cm^{-1}$): 1772, 1676 (C = O).

Beispiel 16

2-(Benzofur-3-yl)-2-oxo-essigsäurebutylester

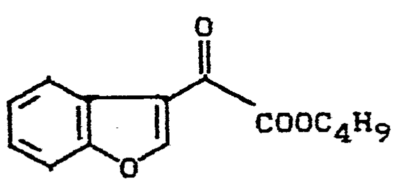

14,9 g Benzofur-3-yl-glykolsäurebutylester und 26,6 g Bleitetraacetat werden 8 h in 125 ml abs. Benzol zum Rückfluß erhitzt. Nach Aufarbeitung wie im Beispiel 15 beschrieben, erhält man 8,7 g (59 % d.Th.) Öl, Sdp. 132-136°C (1 Torr), $n_D^{20}$ = 1.537

$^1$H-NMR (80 MHz, $CDCl_3$, δ-Werte): 8.88 (s, 1H); 8.28 (m, 1H); 7.43 (m, 3H); 4.38 (t, 2H, J = 6,5 Hz); 2.00-1.45 (m, 4H); 0,98 (t, 3H, J = 7 Hz).

Beispiel 17

(Vorprodukt zur Verbindung des Beispiels 16)

Benzofur-3-yl-glykolsäurebutylester

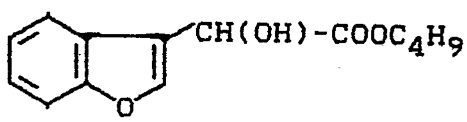

Zu einer Lösung von 49,6 g 3,4-Benzo-6-butoxycarbonyl-2,7-dioxabicyclo[3.2.0]hept-3-en in 340 ml abs. Ether tropft man eine Lösung von 0,7 g p-Toluolsulfonsäure in 340 ml abs. Ether. Nach 48 h Aufbewahren bei Raumtemperatur neutralisiert man mit Triethylamin, filtriert und engt ein. Vakuumdestillation ergibt 25,3 g Benzofur-3-yl-glykolsäurebutylester als Öl, Sdp. 145-150°C (0,7 Torr), $n_D^{20}$ = 1.5308.

$^1$H-NMR (80 MHz, $CDCl_3$, δ-Werte): 7.85-7.10 (m, 5H); 7,70 (s, 1H); 5.41 (s, 1H); 4.18 (t, 2H, J = 7 Hz); 3.66 (s, 1H); 1.80-0.98 (m, 4H); 0.98 (t, 3H, J = 7 Hz).
IR (Kap. Film, ν in $cm^{-1}$): 3475 (O-H), 1740 (C=O).

Beispiel 18

(Vorprodukt zur Verbindung des Beispiels 17)

3,4-Benzo-6-butoxycarbonyl-2,7-dioxabicyclo[3.2.0]hept-3-en

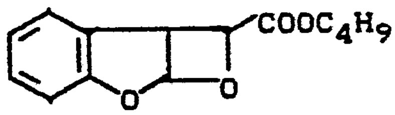

Eine Lösung von 26,0 g Glyoxylsäurebutylester und 11,8 g Benzofuran in 150 ml Benzol wird 60 h bei Raumtemperatur unter Stickstoff bestrahlt. Als Strahlungsquelle verwendet man den Quecksilberhochdruckbrenner Philips HPK 125 W in einem wassergekühlten Schacht aus Pyrex-Glas. Nach Abdestillieren der flüchtigen Komponente isoliert man das Produkt durch Destillation im Hochvakuum. 5 g 3,4-Benzo-2,7-dioxabicyclo[3.2.0]hept-3-en als Öl, Sdp. 140-144°C (0,4 Torr), $n_D^{20}$ = 1.508.

$^1$H-NMR (80 MHz, $CDCl_3$, δ-Werte): 7,45-6,80 (m, 4H); 6.63 (d, 1H, J = 4,0 Hz); 4.87 (d, 1H, J = 3.0 Hz), 4.33 (t, 1H, J = 3,0 Hz); 4.28 (t, 2H, J = 7,0 Hz); 1.95-1.13 (m, 4H); 0,98 (t, 3H, J = 7,0 Hz).

Anwendungsbeispiel

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge an Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine gute Wirksamkeit zeigen in diesem Test z.B. die Verbindungen gemäß den Herstellungsbeispielen: 8, 1, 9, 7, 5 und 12.

Patentansprüche

1. Carbamoyloxythiophen-Derivate der allgemeinen Formel (I)

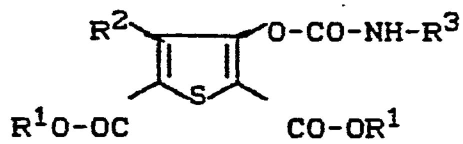

(I)

in welcher

$R^1$ für Alkyl, Alkoxalkyl, Alkylthioalkyl, Fluoralkyl, Cyanalkyl, Alkenyl, Alkinyl oder Cycloalkyl steht,

$R^2$ für Alkyl, gegebenenfalls substituiertes Furyl oder für gegebenenfalls substituiertes Phenyl steht,

$R^3$ für aliphatisches oder carbocyclisches Cyanalkyl, den Rest 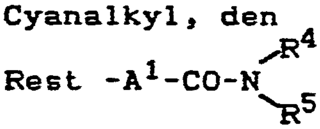 oder den Rest $-A^2-CO-OR^6$

steht, wobei

$A^1$ für einen Alkylenrest steht,

$A^2$ für einen Alkylenrest steht oder eine direkte Bindung bedeutet,

$R^4$ und $R^5$ unabhängig voneinander für Alkyl stehen oder

$R^4$ und $R^5$ gemeinsam mit dem Stickstoffatom, an dem sie stehen, einen heterocyclischen Ring bilden, der weitere Heteroatome enthalten kann und gegebenenfalls substituiert ist und

$R^6$ für Alkyl steht.

2. Carbamoyloxythiophen-Derivate gemäß Anspruch 1, worin in der Formel (I)

$R^1$ für geradkettiges oder verzweigtes Alkyl mit 1 bis 6 Kohlenstoffatomen, für Alkoxyalkyl oder für Alkylthioalkyl jeweils mit 1 bis 5 Kohlenstoffatomen je geradkettigen oder verzweigten Alkylteil, für Fluoralkyl mit 1 bis 4 Kohlenstoffatomen und 1 bis 9 Fluoratomen, für geradkettiges oder verzweigtes Cyanalkyl mit 1 bis 6 Kohlenstoffatomen im Alkylteil, für geradkettiges oder verzweigtes Alkenyl mit 2 bis 5 Kohlenstoffatomen, für geradkettiges oder verzweigtes Alkinyl mit 3 bis 6 Kohlenstoffatomen oder für Cycloalkyl mit 5 bis 7 Kohlenstoffatomen steht,

$R^2$ für geradkettiges oder verzweigtes Alkyl mit 1 bis 5 Kohlenstoffatomen, für gegebenenfalls substituiertes Furyl oder für gegebenenfalls ein- bis fünffach, gleich oder verschieden durch Alkyl mit 1 bis 4 Kohlenstoffatomen, Alkoxy oder Alkylthio mit je 1 bis 4 Kohlenstoffatomen, Halogen, Nitro, Halogenalkyl, Halogenalkoxy oder

Halogenalkylthio mit jeweils 1 bis 4 Kohlenstoffatomen und je 1 bis 5 gleichen oder verschiedenen Halogenatomen substituiertes Phenyl steht,

$R^3$ für geradkettiges oder verzweigtes oder für gesättigtes, cyclisches Cyanalkyl mit 1 bis 6 Kohlenstoffatomen im Alkylteil, für einen Rest

$-A^1-CO-N\begin{matrix}R^4\\R^5\end{matrix}$ oder den Rest $-A^2-CO-OR^6$ steht,

wobei

$A^1$ für Alkylen mit 1 bis 6 Kohlenstoffatomen steht,

$R^4$ und $R^5$ unabhängig voneinander für Alkyl mit 1 bis 5 Kohlenstoffatomen stehen oder

$R^4$ und $R^5$ gemeinsam mit dem Stickstoffatom, an dem sie stehen, einen gesättigten oder ungesättigten, heterocyclischen Ring mit bis zu 6 Kohlenstoffatomen bilden können, der durch weitere Sauerstoffatome unterbrochen sein kann und der gegebenenfalls ein- bis fünffach, gleich oder verschieden durch Alkyl mit 1 bis 4 Kohlenstoffatomen substituiert sein kann,

$A^2$ für Alkylen mit 1 bis 6 Kohlenstoffatomen steht oder eine direkte Bindung bedeutet und

$R^6$ für Alkyl mit 1 bis 10 Kohlenstoffatomen steht.

3. Carbamoyloxythiophen-Derivate gemäß Anspruch 1, wobei in der Formel (I)

$R^1$ für geradkettiges oder verzweigtes Alkyl mit 1 bis 5 Kohlenstoffatomen, für Alkoxyalkyl oder für Alkylthioalkyl jeweils mit 1 bis 4 Kohlenstoffatomen je geradkettigen oder verzweigten Alkylteil, für Fluoralkyl mit 1 oder 2 Kohlenstoffatomen und 1 bis 5 Fluoratomen, für geradkettiges oder verzweigtes Cyanalkyl mit 1 bis 5 Kohlenstoffatomen im Alkylteil, für geradkettiges oder verzweigtes Alkenyl mit 2 bis 4 Kohlenstoffatomen, für geradkettiges oder verzweigtes Alkinyl mit 3 bis 6 Kohlenstoffatomen oder für Cycloalkyl mit 5 oder 6 Kohlenstoffatomen steht,

$R^2$ für geradkettiges oder verzweigtes Alkyl mit 1 bis 5 Kohlenstoffatomen, für Furyl, Benzofuryl oder ein- oder zweifach durch Halogen, Alkyl oder Alkoxy substituiertes Benzofuryl, oder für gegebenenfalls ein- bis dreifach, gleich oder verschieden durch Alkyl mit 1 bis 3 Kohlenstoffatomen, Alkoxy oder Alkylthio mit je 1 bis 3 Kohlenstoffatomen, Fluor, Chlor, Nitro, Halogenalkyl, Halogenalkoxy oder Halogenalkylthio mit jeweils

0243776

1 oder 2 Kohlenstoffatomen und je 1 bis 5 gleichen oder verschiedenen Halogenatomen substituiertes Phenyls steht,

$R^3$ für geradkettiges oder verzweigtes oder für gesättigtes, cyclisches Cyanalkyl mit 1 bis 6 Kohlenstoffatomen im Alkylteil, für einen Rest

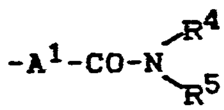 oder den Rest $-A^2-CO-OR^6$ steht,

wobei

$A^1$ für Alkylen mit 1 bis 5 Kohlenstoffatomen steht,

$R^4$ und $R^5$ unabhängig voneinander für Alkyl mit 1 bis 3 Kohlenstoffatomen stehen oder

$R^4$ und $R^5$ gemeinsam mit dem Stickstoffatom, an dem sie stehen, einen gesättigten oder ungesättigten, heterocyclischen Ring mit bis zu 6 Kohlenstoffatomen bilden können, der durch ein weiteres Sauerstoffatom unterbrochen sein kann und der gegebenenfalls ein-bis dreifach, gleich oder verschieden durch Methyl oder Ethyl substituiert sein kann,

$A^2$ für Alkylen mit 1 bis 5 Kohlenstoffatomen steht oder eine direkte Bindung bedeutet und

$R^6$ für Alkyl mit 1 bis 8 Kohlenstoffatomen steht.

4. Carbamoyloxythiophen-Derivate gemäß Anspruch 1, wobei in der Formel (I)

$R^1$ für Methyl, Ethyl, n- oder iso-Propyl, 2,2-Dimethylpropyl, 2-Methoxyethyl, 2-Ethoxyethyl, Methoxypropyl, 2-Methylthioethyl, 2-Ethylthioethyl, 2,2,2-Trifluorethyl, Cyanmethyl, 2-Cyanethyl, Allyl, Methallyl, 3-Propinyl, 1,1-Dimethyl-3-propinyl oder Cyclopentyl steht,

$R^2$ für Methyl, Ethyl, n-Propyl, iso-Propyl, n-Butyl, sek.-Butyl, iso-Butyl, tert.-Butyl, Furyl, Phenyl oder für ein- bis dreifach, gleich oder verschieden durch Methyl, Ethyl, Fluor und Chlor substituiertes Phenyl steht,

$R^3$ für geradkettiges oder verzweigtes Cyanalkyl mit 2 bis 6 Kohlenstoffatomen im Alkylteil, für gesättigtes, cyclisches Cyanalkyl mit 5 oder 6 Kohlenstoffatomen im Ring, für den Rest

$$-A^1-CO-N\begin{matrix}R^4\\R^5\end{matrix}$$ oder den Rest $-A^2-CO-OR^6$ steht,

wobei

$A^1$ für Alkylen mit 1 bis 5 Kohlenstoffatomen steht,

$R^4$ und $R^5$ unabhängig voneinander für Methyl oder Ethyl stehen oder

$R^4$ und $R^5$ zusammen mit dem Stickstoffatom, an dem sie stehen, einen 6-Ring bilden, der durch ein Sauerstoffatom unterbrochen sein kann und der gegebenenfalls ein- bis dreifach, gleich oder verschieden durch Methyl oder Ethyl substituiert sein kann,

$A^2$ für Alkylen mit 1 bis 5 Kohlenstoffatomen steht oder eine direkte Bindung bedeutet und

$R^6$ für Alkyl mit 1 bis 8 Kohlenstoffatomen steht.

5. Carbamoyloxythiophen-Derivate gemäß Anspruch 1, wobei in der Formel (I)

$R^1$ für Methyl, Ethyl, Isopropyl, n-Propyl, sek.-Butyl, 2,2-Dimethylpropyl oder Cyclopentyl steht,

$R^2$ für Methyl, Isopropyl, Furyl oder Phenyl steht,

$R^3$ für 1-Cyan-1-methylethyl, 2-Cyanethyl, 5-Cyanpentyl oder für den Rest $-A^2-COOR^6$ steht, wobei

$A^2$ eine direkte Bindung oder ein Pentylenrest bedeutet und

$R^6$ Methyl, Ethyl, Isopropyl, Butyl, 2,2-Dimethylpropyl, 2-Methylhexyl oder Octyl bedeutet.

6. Verfahren zur Herstellung von Carbamoyloxythiophen-Derivaten der allgemeinen Formel (I)

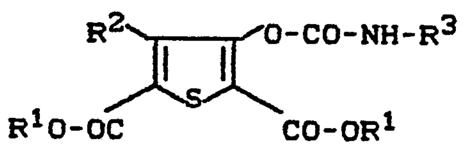

(I)

in welcher

$R^1$ für Alkyl, Alkoxalkyl, Alkylthioalkyl, Fluoralkyl, Cyanalkyl, Alkenyl, Alkinyl oder Cycloalkyl steht,

$R^2$ für Alkyl, gegebenenfalls substituiertes Furyl oder für gegebenenfalls substituiertes Phenyl steht,

$R^3$ für aliphatisches oder carbocyclisches Cyanalkyl, den Rest $-A^1-CO-N(R^4)(R^5)$ oder den Rest $-A^2-CO-OR^6$

steht, wobei

$A^1$ für einen Alkylenrest steht,

$A^2$ für einen Alkylenrest steht oder eine direkte Bindung bedeutet,

$R^4$ und $R^5$ unabhängig voneinander für Alkyl stehen oder

$R^4$ und $R^5$ gemeinsam mit dem Stickstoffatom, an dem sie stehen, einen heterocyclischen Ring bilden, der weitere Heteroatome enthalten kann und gegebenenfalls substituiert ist und

$R^6$ für Alkyl steht,

dadurch gekennzeichnet, daß man

a) ein Isocyanat der allgemeinen Formel (II)

$$OCN\text{-}R^3 \qquad (II)$$

in welcher

$R^3$ die oben angegebene Bedetung hat,

mit Hydroxythiophen-Derivaten der allgemeinen Formel (III)

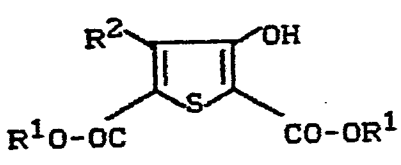

(III)

in welcher

$R^1$ und $R^2$ die oben angegebenen Bedeutungen haben,

gegebenenfalls in Gegenwart eines Lösungs- oder Verdünnungsmittels und gegebenenfalls in Gegenwart einer katalytisch wirkenden Substanz umsetzt oder

b) ein 4-Hydroxythiophen-Derivat der Formel (III) in einer ersten Stufe mit Phosgen in Gegenwart eines Chlorwasserstoff-bindenden Mittels zu einem 4-Chlorcarbonyloxythiophen-Derivat der Formel (IV)

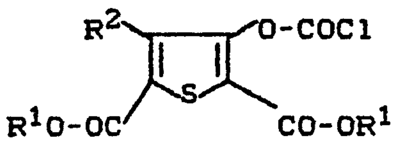

(IV)

in der $R^1$ und $R^2$ die oben angegebene Bedeutung haben,

umsetzt und dann in einer zweiten Stufe das 4-Chlorcarbonyloxythiophen-Derivat der Formel (IV) mit einem Amin der Formel (V)

$$NH_2-R^3 \quad (V)$$

in der $R^3$ die oben angegebene Bedeutung hat,

oder mit einem der Formel (V) entsprechenden Salz in Gegenwart eines säurebindenden Mittels umsetzt.

7. Schädlingsbekämpfungsmittel, gekennzeichnet durch einen Gehalt an mindestens einem Carbamoyloxythiophen-Derivat der Formel (I) gemäß den Ansprüchen 1 bis 6.

8. Verfahren zur Bekämpfung von Schädlingen, dadurch gekennzeichnet, daß man Carbamoyloxythiophen-Derivate der Formel (I) gemäß den Ansprüche 1 bis 6 auf Schädlinge und/oder ihren Lebensraum einwirken läßt.

9. Verwendung von Carbamoyloxythiophen-Derivaten der Formel (I) gemäß den Ansprüchen 1 bis 6 zur Bekämpfung von Schädlingen.

10. Verfahren zur Herstellung von Schädlingsbekämpfungsmitteln, dadurch gekennzeichnet, daß man Carbamoyloxythiophen-Derivate der Formel (I) gemäß den Ansprüchen 1 bis 6 mit Streckmitteln vermischt.

11. Hydroxythiophenderivate der allgemeinen Formel (IIIa)

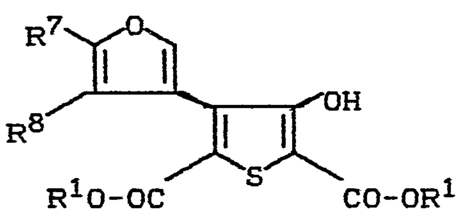

(IIIa)

worin $R^1$ die in Anspruch 1 angegebene Bedeutung hat und $R^7$ und $R^8$ Wasserstoff bedeuten oder gemeinsam mit dem Furylrest, an den sie gebunden sind, einen Benzofurylrest der Formel (VII)

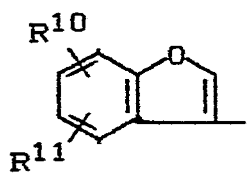

(VII)

darstellen, worin $R^{10}$ und $R^{11}$ unabhängig voneinander Wasserstoff, Halogen, Alkyl oder Alkoxy bedeuten.

12. Verfahren zur Herstellung von Verbindungen der allgemeinen Formel (IIIa)

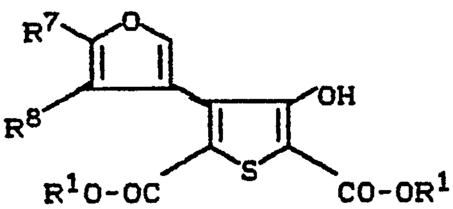

(IIIa)

worin $R^1$, $R^7$ und $R^8$ die in Anspruch 11 angegebene Bedeutung haben,

dadurch gekennzeichnet, daß man einen Thiodiessigsäureester der allgemeinen Formel

$$R^1O-OC-CH_2-S-CH_2-CO-OR^1$$

worin $R^1$ die oben angegebene Bedeutung hat, mit einem 2-(Fur-3-yl)-2-oxo-essigsäureester der allgemeinen Formel

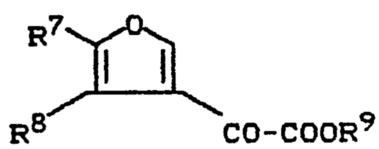

(VIII)

in der $R^7$ und $R^8$ die in Formel IIIa in Anspruch 11 angegebene Bedeutung haben, und $R^9$ einen Alkylrest bedeutet, in Gegenwart von Kalium-tert.-butanolat umsetzt und anschließend in Säure gießt.

13. 2-(Fur-3-yl)-2-oxo-essigsäureester der allgemeinen Formel (VIII)

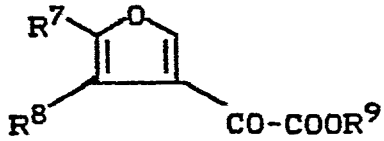

(VIII)

worin $R^7$ und $R^8$ Wasserstoff bedeuten oder gemeinsam mit dem Furylrest, an den sie gebunden sind, einem Benzofurylrest der allgemeinen Formel

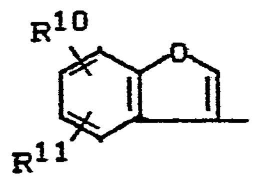

(VII)

darstellen, worin $R^{10}$ und $R^{11}$ unabhängig voneinander Wasserstoff, Halogen, Alkyl oder Alkoxy bedeuten, und

worin $R^9$ für gegebenenfalls durch Halogen oder Alkoxy substituiertes Alkyl steht.

14. Verfahren zur Herstellung von 2-(Fur-3-yl)-2-oxo-essigsäureestern der allgemeinen Formel (VIII)

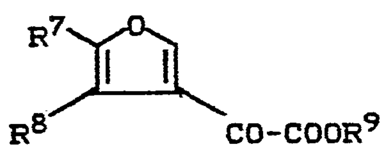

(VIII)

worin $R^7$, $R^8$ und $R^9$ die in Anspruch 13 angegebene Bedeutung haben,

dadurch gekennzeichnet, daß man Fur-3-yl-glykolsäureester der allgemeinen Formel (VI)

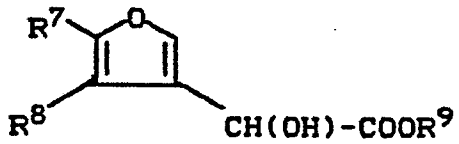

(VI)

worin $R^7$, $R^8$ und $R^9$ die oben angegebene Bedeutung haben, mit Oxidationsmitteln in einem inerten organischen Lösungsmittel umsetzt.

15. Benzofur-3-ylglykolsäureester der allgemeinen Formel (VIa)

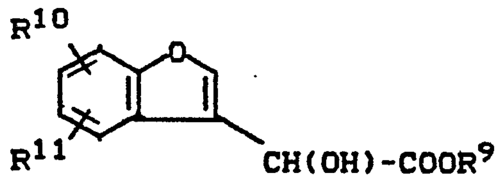

(VIa)

worin $R^9$ für gegebenenfalls durch Halogen oder Alkoxysubstituiertes Alkyl steht und $R^{10}$ und $R^{11}$ unabhängig voneinander Wasserstoff, Halogen, Alkyl oder Alkoxy bedeuten.

16. Verfahren zur Herstellung von Benzofur-3-yl-glykolsäureestern der allgemeinen Formel (IVa)

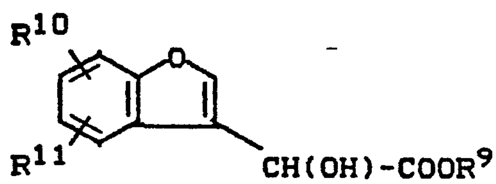

(VIa)

worin $R^9$, $R^{10}$ und $R^{11}$ die in Anspruch 15 angegebene Bedeutung haben,

0243776

dadurch gekennzeichnet, daß man Glyoxylsäureester der allgemeinen Formel $OHC-COOR^9$, worin $R^9$ die in Anspruch 15 angegebene Bedeutung hat, an Benzofurane der allgemeinen Formel

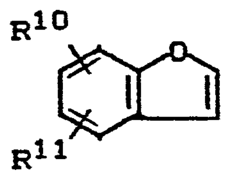

worin $R^{10}$ und $R^{11}$ die oben angegebene Bedeutung haben, photochemisch anlagert und die erhaltenen 2,7-Dioxabicyclo[3.2.0]hept-3-ene mit Säure isomerisiert.